# EUROPEAN PATENT APPLICATION

(11) **EP 3 838 114 A1**
(43) Date of publication of application: **23.06.2021**
(21) Application number: 20215153.6
(22) Date of filing: 17.12.2020
(51) Int. Cl.: A61B 3/117, A61B 3/00, A61B 3/10

(54) **CRYSTALLINE LENS DATA OBTAINING METHOD, OPHTHALMOLOGIC DEVICE, AND PROGRAM FOR OPHTHALMOLOGIC DEVICE**

(30) Priority: 18.12.2019 JP 2019228036
(71) Applicant: Chukyo Medical Co., Inc., Nagoya-shi, Aichi 456-0032 (JP)
(72) Inventor: MAJIMA, Kiyoyuki, Nagoya-shi Aichi, 454-0843 (JP); ICHIKAWA, Kazuo, Nagoya-shi Aichi, 456-00332 (JP); ICHIKAWA, Kei, Nagoya-shi Aichi, 456-00332 (JP)
(74) Representative: Ter Meer Steinmeister & Partner

(57) **Abstract**

Provided are a method, a device, or a program for easily determining a crystalline lens nucleus state.

A gray scale anterior segment image taken by optical coherence tomography is obtained (S21), and posterization of the anterior segment image is performed to obtain tone-changed images having 3 tones, 4 tones, and 5 tones (S22). One, of a plurality of predetermined grades, corresponding to the crystalline lens nucleus state is determined based on a combination of states of observed images of the crystalline lens nucleus in the tone-changed images (S23). The grades are set for classification according to whether or not an image of a crystalline lens nucleus is observed, whether the observed image is spot-shaped or band-shaped, or whether or not the entire image of the crystalline lens nucleus is observed.

## Description

### BACKGROUND OF THE INVENTION

### Field of the Invention

This disclosure relates to a technique for determining a crystalline lens nucleus state.

### Description of Related Art

To date, a method for performing classification (in other words, grading) of a color, transparency, hardness, or the like of a crystalline lens nucleus based on an image of the crystalline lens measured by a slit-lamp microscope or the like in order to perform examination for a cataract or determine a cataract surgery method for an eye diagnosed as having a cataract, has been known (for example, see: "Lens Opacities Classification System II (LOCS II)", Arch Ophthalmol, July 1989, Vol 107, p.991-997; "The Lens Opacities Classification System III", Arch Ophthalmol, June 1993, Vol 111, p.831-836; "Reproducibility of the Wisconsin cataract grading system in the Blue Mountains Eye Study", Ophthalmic Epidemiology, 1997, Vol.4, No.3, p.119-126; "Use of Photographic Techniques to Grade Nuclear Cataract" Investigative Ophthalmology & Visual Science, January 1988, Vol.29, No.1, p.73-77; "The Oxford Clinical Cataract Classification and Grading System", International Ophthalmology, 1986, Vol.9, p.207-225; "Classification System for Cataracts", Ophthalmic Res, 1990, Vol.22, p.46-50; and "Emery kaku koudo bunrui no saibunka" Rinsho Ganka, 1996-nen 6-gatsu, 50-kan, 6-gou, p. 1087-1090 (Modification of grading system by Emery-Little for nuclear hardness, Japanese Journal of Clinical Ophthalmology, June, 1996, Volume 50, Issue 6, pp. 1087-1090)). In a conventional method, reference images based on grades of a color, transparency, or the like of a crystalline lens nucleus are provided, and an image of a crystalline lens to be determined and the reference images are compared with each other, thereby determining a grade corresponding to the color, transparency, or the like of the nucleus of the crystalline lens to be determined.

In a conventional method, in a case where, among a plurality of predetermined grades, a grade corresponding to a color, transparency, or the like of a crystalline lens nucleus in an image is determined, it is not easy to distinguish the color, transparency, or the like of the crystalline lens nucleus from that in a different grade. As a result, determination of the crystalline lens nucleus state becomes difficult, and reproducibility of the crystalline lens nucleus state having been determined is low.

This disclosure has been made in view of the above-mentioned problems, and an object of this disclosure is to provide a method for obtaining data that allows a crystalline lens nucleus state to be easily determined, an ophthalmologic device capable of obtaining data that allows a crystalline lens nucleus state to be easily determined, and a program, for an ophthalmologic device, capable of obtaining data that allows a crystalline lens nucleus state to be easily determined.

### SUMMARY OF THE INVENTION

In order to solve the aforementioned problems, a crystalline lens data obtaining method according to this disclosure includes: an obtaining step of obtaining an image of a crystalline lens; and a tone changing step of lowering the number of tones of an original image that is the image obtained in the obtaining step.

An ophthalmologic device according to this disclosure includes an obtaining portion configured to obtain an image of a crystalline lens; and a tone changing portion configured to lower the number of tones of an original image that is the image obtained by the obtaining portion.

A program for an ophthalmologic device stored therein, the program causing a computer to function as: an obtaining portion configured to obtain an image of a crystalline lens; and a tone changing portion configured to lower the number of tones of an original image that is the image obtained by the obtaining portion.

According to this disclosure, the number of tones of an original image of a crystalline lens is lowered in the tone changing step or by the tone changing portion, whereby a state of a crystalline lens nucleus in the image can be emphasized. Thus, crystalline lens data that allows a crystalline lens nucleus state to be easily determined can be obtained.

### BRIEF DESCRIPTION OF THE DRAWINGS

FIG. 1 illustrates a configuration of an ophthalmological examination system;
FIG. 2 schematically illustrates a tomographic image of an anterior segment;
FIG. 3 is a flow chart showing a procedure for setting a precondition for classifying crystalline lens nucleus states into a plurality of grades;
FIG. 4 is a flow chart showing a specific procedure of step S3 in FIG. 3;
FIG. 5 is a flow chart showing a procedure for classifying a crystalline lens nucleus state, according to a first embodiment;
FIG. 6 is a flow chart showing a specific procedure of step S23 in FIG. 5;
FIG. 7 illustrates an original image of an anterior segment and also illustrates an anterior segment image to be classified as a grade of nucleus classification number "1";
FIG. 8 illustrates an original image of an anterior segment and also illustrates an anterior segment image to be classified as a grade of nucleus classification number "2";
FIG. 9 illustrates an original image of an anterior segment and also illustrates an anterior segment image to be classified as a grade of nucleus classification number "3a";
FIG. 10 illustrates an original image of an anterior segment and also illustrates an anterior segment image to be classified as a grade of nucleus classification number "3b";
FIG. 11 illustrates an original image of an anterior segment and also illustrates an anterior segment image to be classified as a grade of nucleus classification number "4";
FIG. 12 illustrates an original image of an anterior segment and also illustrates an anterior segment image to be classified as a grade of nucleus classification number "5";
FIG. 13 illustrates an anterior segment image, taken by a slit-lamp microscope, corresponding to the anterior segment image in FIG. 7:
FIG. 14 illustrates an anterior segment image, taken by a slit-lamp microscope, corresponding to the anterior segment image in FIG. 8;
FIG. 15 illustrates an anterior segment image, taken by a slit-lamp microscope, corresponding to the anterior segment image in FIG. 9;
FIG. 16 illustrates an anterior segment image, taken by a slit-lamp microscope, corresponding to the anterior segment image in FIG. 10;
FIG. 17 illustrates an anterior segment image, taken by a slit-lamp microscope, corresponding to the anterior segment image in FIG. 11;
FIG. 18 illustrates an anterior segment image, taken by a slit-lamp microscope, corresponding to the anterior segment image in FIG. 12;
FIG. 19 illustrates an image obtained by changing the number of tones of the anterior segment image in FIG. 7 to 3;
FIG. 20 illustrates an image obtained by changing the number of tones of the anterior segment image in FIG. 7 to 4;
FIG. 21 illustrates an image obtained by changing the number of tones of the anterior segment image in FIG. 7 to 5;
FIG. 22 illustrates an image obtained by changing the number of tones of the anterior segment image in FIG. 8 to 3;
FIG. 23 illustrates an image obtained by changing the number of tones of the anterior segment image in FIG. 8 to 4;
FIG. 24 illustrates an image obtained by changing the number of tones of the anterior segment image in FIG. 8 to 5;
FIG. 25 illustrates an image obtained by changing the number of tones of the anterior segment image in FIG. 9 to 3;
FIG. 26 illustrates an image obtained by changing the number of tones of the anterior segment image in FIG. 9 to 4;
FIG. 27 illustrates an image obtained by changing the number of tones of the anterior segment image in FIG. 9 to 5;
FIG. 28 illustrates an image obtained by changing the number of tones of the anterior segment image in FIG. 10 to 3;
FIG. 29 illustrates an image obtained by changing the number of tones of the anterior segment image in FIG. 10 to 4;
FIG. 30 illustrates an image obtained by changing the number of tones of the anterior segment image in FIG. 10 to 5;
FIG. 31 illustrates an image obtained by changing the number of tones of the anterior segment image in FIG. 11 to 3;
FIG. 32 illustrates an image obtained by changing the number of tones of the anterior segment image in FIG. 11 to 4;
FIG. 33 illustrates an image obtained by changing the number of tones of the anterior segment image in FIG. 11 to 5;
FIG. 34 illustrates an image obtained by changing the number of tones of the anterior segment image in FIG. 12 to 3;
FIG. 35 illustrates an image obtained by changing the number of tones of the anterior segment image in FIG. 12 to 4;
FIG. 36 illustrates an image obtained by changing the number of tones of the anterior segment image in FIG. 12 to 5;
FIG. 37 illustrates an image obtained by changing the number of tones of the anterior segment image in FIG. 10 to 6;
FIG. 38 illustrates an image obtained by changing the number of tones of the anterior segment image in FIG. 10 to 7;
FIG. 39 illustrates an image obtained by changing the number of tones of the anterior segment image in FIG. 10 to 8;
FIG. 40 illustrates an image obtained by changing the number of tones of the anterior segment image in FIG. 11 to 6;
FIG. 41 illustrates an image obtained by changing the number of tones of the anterior segment image in FIG. 11 to 7;
FIG. 42 illustrates an image obtained by changing the number of tones of the anterior segment image in FIG. 11 to 8;
FIG. 43 illustrates an image obtained by changing the number of tones of the anterior segment image in FIG. 12 to 6;
FIG. 44 illustrates an image obtained by changing the number of tones of the anterior segment image in FIG. 12 to 7;
FIG. 45 illustrates an image obtained by changing the number of tones of the anterior segment image in FIG. 12 to 8; and
FIG. 46 is a flow chart showing a procedure for classifying a crystalline lens nucleus state, according to a second embodiment.

### DESCRIPTION OF THE PREFERRED EMBODIMENTS

### (First embodiment)

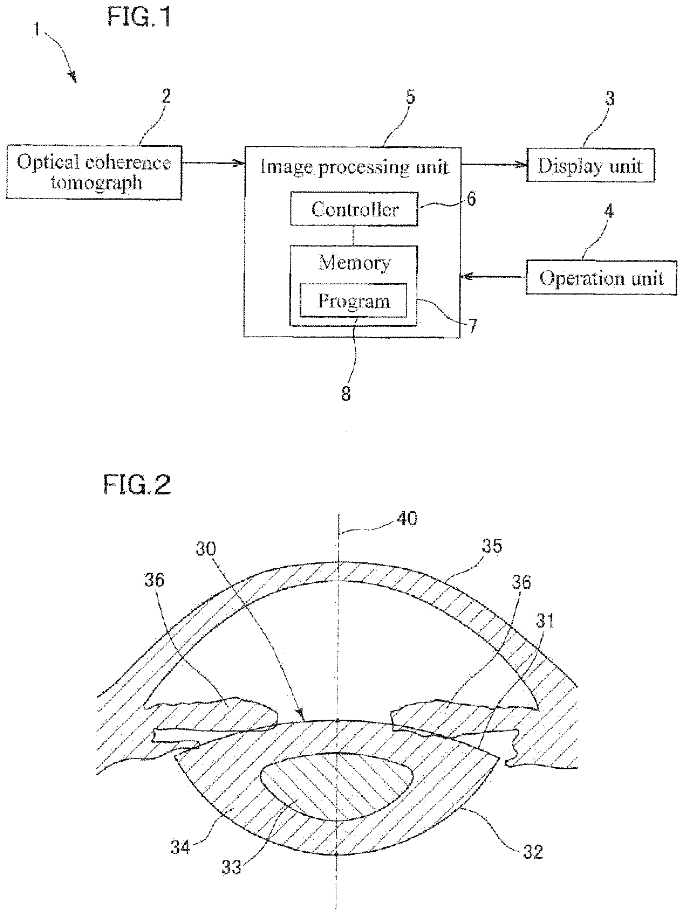

A first embodiment of this disclosure will be described below with reference to the drawings. FIG. 1 illustrates a configuration of an ophthalmological examination system 1. The ophthalmological examination system 1 obtains data for determining a hardness of a crystalline lens nucleus, as a crystalline lens nucleus state, and presents (displays) the data. The ophthalmological examination system 1 includes an optical coherence tomograph 2, a display unit 3, an operation unit 4, and an image processing unit 5.

The optical coherence tomograph 2 takes a tomographic image of an anterior segment by means of optical coherence tomography (OCT). The optical coherence tomograph 2 splits light from a low-coherence light source into reference light and measurement light to be applied to a subject eye, and obtains a tomographic image of an anterior segment based on coherent light between the reference light and reflected light obtained by reflecting the measurement light by the subject eye. The optical coherence tomograph 2 is a frequency swept source OCT (SS-OCT) that uses, for example, a wavelength swept laser as a light source, and performs Fourier transform of an obtained spectrum to obtain depth information.

The optical coherence tomograph 2 outputs a gray scale image as a tomographic image of an anterior segment. The number of tones of the image is, for example, 256. In the gray scale image represented by 256 tones, any value ranging from "0" to "255" is given as a tone value of a pixel. The tone value of "0" represents black and the tone value of "255" represents white. The tone values of "1" to "254" represent gray colors having different color depths (gradation). The greater the tone value of the gray color is, the closer the gray color is to white.

FIG. 2 schematically illustrates a tomographic image of an anterior segment which is taken by the optical coherence tomograph 2. The tomographic image in FIG. 2 represents an image of the anterior segment cut at a plane that is almost parallel to a reference line 40 (visual axis, optical axis, or the like) extending in the depth direction of the anterior segment. The tomographic image includes images of parts of the anterior segment such as a crystalline lens 30, a cornea 35, and irises 36. The crystalline lens 30 includes an anterior capsule 31, a posterior capsule 32, a crystalline lens nucleus 33, and a crystalline lens cortex 34. The crystalline lens nucleus 33 is located at the center portion of the crystalline lens 30. The crystalline lens cortex 34 is located between the crystalline lens nucleus 33 and the crystalline lens capsules 31, 32. In FIG. 2, the parts 31 to 34 of the crystalline lens 30 are clearly indicated for easy understanding. However, the parts 31 to 34 may be unclear in an actual image or it may be difficult to distinguish the parts 31 to 34 from each other.

Returning to FIG. 1, the display unit 3 is a display such as a liquid crystal display for displaying, for example, an anterior segment image outputted by the image processing unit 5. The operation unit 4 is operated by a user such as a person (doctor or the like) who evaluates an anterior segment. The operation unit 4 may be implemented by a keyboard, a mouse, or the like, or a touch panel provided on a screen of the display unit 3. The operation unit 4 is used for, for example, indicating contents (specifically, for example, the number of tones to which the number of tones of an image is to be changed in posterization described below) of a process to be performed by the image processing unit 5.

The image processing unit 5 obtains an anterior segment image outputted by the optical coherence tomograph 2, and performs image processing of the obtained anterior segment image. In this embodiment, the image processing unit 5 is connected directly to the optical coherence tomograph 2. However, the image processing unit 5 may not necessarily be connected directly to the optical coherence tomograph 2. In a case where the image processing unit 5 and the optical coherence tomograph 2 are not connected directly to each other, for example, image data outputted by the optical coherence tomograph 2 is stored in a portable memory (such as a USB memory), and the memory is connected to the image processing unit 5, whereby the image processing unit 5 may read the image data stored in the memory.

The image processing unit 5 includes a controller 6 and a non-volatile memory 7 such as a ROM. For example, a program 8 for a process performed by the controller 6 is stored in the memory 7. The controller 6 is implemented by a CPU or the like, and performs processing according to the program 8 stored in the memory 7. Specifically, the controller 6 performs posterization for lowering a tone of an anterior segment image, and causes the display unit 3 to display an image having been subjected to the posterization. The memory 7 is a non-transitory tangible storage medium for storing computer-readable program and data in a non-transitory manner. The non-transitory tangible storage medium is implemented by a semiconductor memory, a magnetic disk, or the like.

In this embodiment, the ophthalmological examination system 1 is used for determining a hardness of a crystalline lens nucleus in order to determine a difficulty level or a method of a cataract surgery for an eye to be subjected to the cataract surgery (in other words, a crystalline lens diagnosed as a cataract). In the cataract surgery, an opacifying whitish crystalline lens is removed, and an intraocular lens is implanted in the anterior segment. Kinds of the cataract surgery include phacoemulsification and aspiration (PEA), extracapsular cataract extraction (ECCE), and intracapsular cataract extraction (ICCE). In the phacoemulsification and aspiration, an anterior capsule is opened, and a nucleus and a cortex are pulverized by ultrasonic waves, and aspirated. In the extracapsular cataract extraction, the anterior capsule is opened, and a nucleus and a cortex are extracted. In the intracapsular cataract extraction, the entire crystalline lens is extracted.

In a case where phacoemulsification and aspiration is performed as the cataract surgery, the difficulty level of the surgery is higher for a hard crystalline lens nucleus. In this case, the phacoemulsification and aspiration performed by a less-experienced doctor may cause a serious complication. Therefore, a well-experienced doctor preferably performs the phacoemulsification and aspiration. Ultrasonic output may be adjusted in the phacoemulsification and aspiration so as to correspond to a hardness of a crystalline lens nucleus. If the crystalline lens nucleus is hard, for example, extracapsular cataract extraction may be performed instead of the phacoemulsification and aspiration. Thus, it is useful to determine a hardness of a crystalline lens nucleus of an eye to be subjected to cataract surgery.

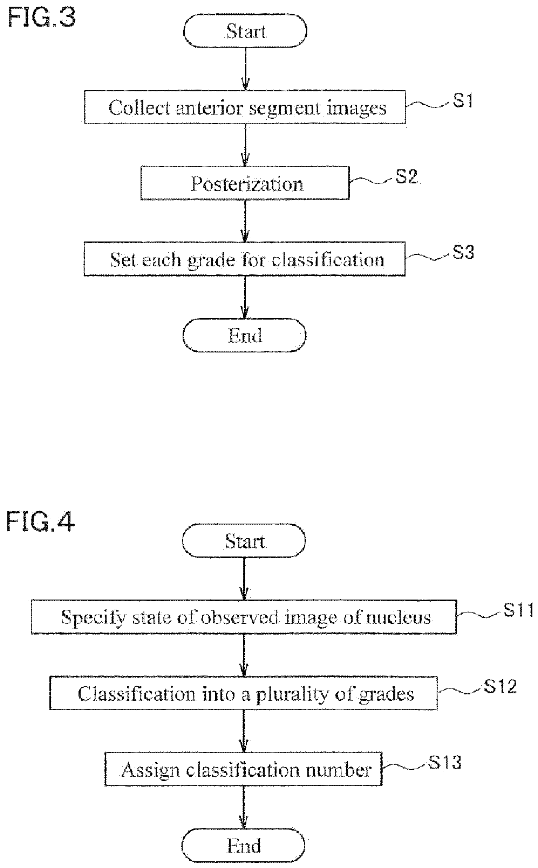

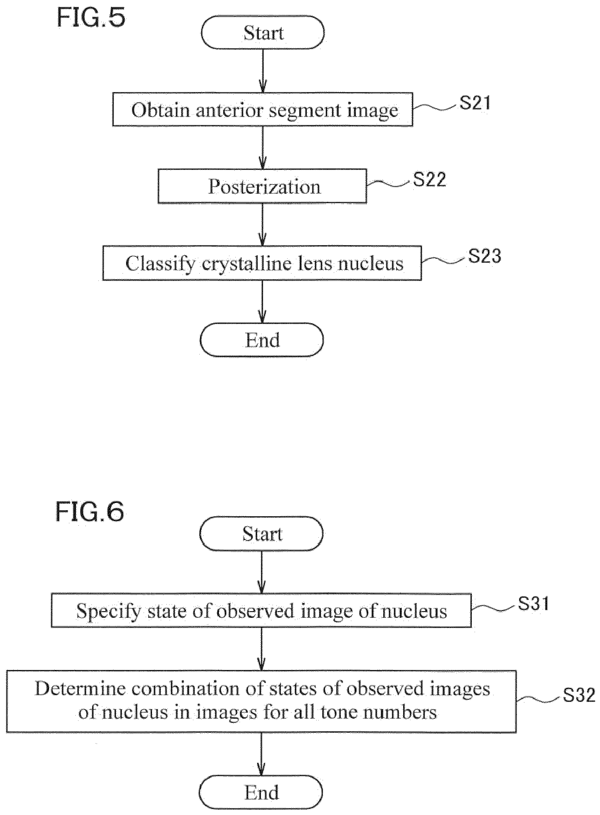

Thus, in this embodiment, hardnesses of crystalline lens nuclei are classified according to the procedure shown in FIG. 3 to FIG. 6. FIG. 3 and FIG. 4 show a procedure for setting a precondition for classifying hardnesses of crystalline lens nuclei into a plurality of grades. That is, the number of grades into which the hardnesses are classified and a criterion for each grade are set in the procedure. FIG. 5 and FIG. 6 show a procedure for determining, among a plurality of grades set in FIG. 3 and FIG. 4, a grade corresponding to a state (hardness) of a crystalline lens nucleus of a crystalline lens to be determined, under the precondition that the grades into which the hardnesses of the nuclei are to be classified have been set as shown in FIG. 3 and FIG. 4. The procedure shown in FIG. 3 and FIG. 4 will be firstly described.

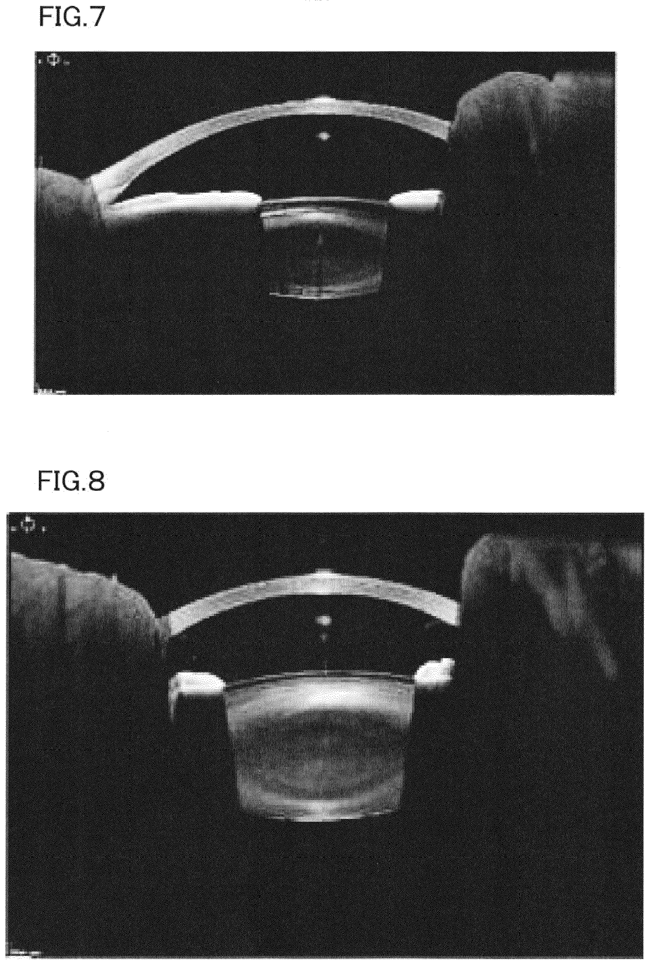

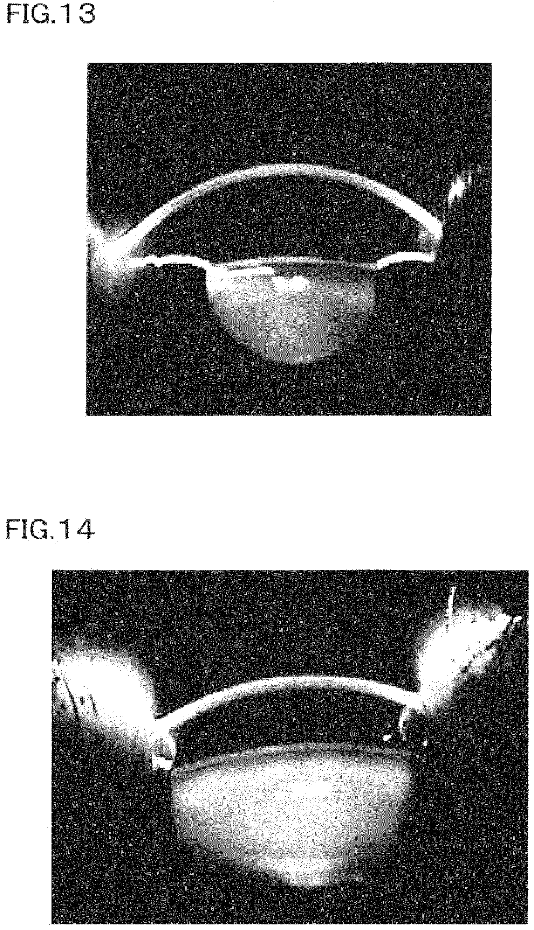

In FIG. 3, a plurality of anterior segment images are firstly collected (S1). The plurality of anterior segment images to be collected are images of different eyes each of which has been diagnosed as having a cataract, and are also gray scale tomographic images taken by optical coherence tomography (the optical coherence tomograph 2). Furthermore, images, obtained by optical coherence tomography, of eyes having different crystalline lens nucleus colors and transparencies in color images of anterior segments that are obtained by a slit-lamp microscope or the like, are preferably collected as the plurality of anterior segment images to be collected. FIG. 7 to FIG. 12 illustrate a part of anterior segment images collected in step S1. The images shown in FIG. 7 to FIG. 12 are images taken by an optical coherence tomograph "ANTERION (registered trademark)" available from Heidelberg Engineering. The "ANTERION (registered trademark)" is a frequency swept source OCT (SS-OCT). In the "ANTERION (registered trademark)", a wavelength of laser is 1300 nm. FIG. 13 to FIG. 18 show anterior segment images, obtained by a slit-lamp microscope, corresponding to the anterior segment images shown in FIG. 7 to FIG. 12, respectively.

Subsequently, the image processing unit 5 reads each of the anterior segment images collected in step S1, and the image processing unit 5 performs a process (posterization) of lowering the number of tones of each anterior segment image (S2). In this embodiment, the image processing unit 5 performs the posterization such that original images are anterior segment images each having 256 tones, and, for each original image, 256 tones are reduced to 3 tones to form a 3 tone image, 256 tones are reduced to 4 tones to form a 4 tone image, and 256 tones are reduced to 5 tones to form a 5 tone image. The image processing unit 5 causes the display unit 3 to display the 3 tone image, the 4 tone image, and the 5 tone image which are obtained by the posterization or causes a printer (not shown) to print the images. That is, the image processing unit 5 presents the 3 tone image, the 4 tone image, and the 5 tone image (presenting step).

For example, a tone value of each pixel of the original image is converted as indicated in Table 1 in order to obtain the 3 tone image from the original image. For example, a tone value of each pixel of the original image is converted as indicated in Table 2 in order to obtain the 4 tone image from the original image. For example, a tone value of each pixel of the original image is converted as indicated in Table 3 in order to obtain the 5 tone image from the original image.

**[Table 1]**

| | | | |
|---|---|---|---|
| Original tone value | 0-85 | 86-170 | 171-255 |
| Converted tone value | 0 | 127 | 255 |

**[Table 2]**

| | | | | |
|---|---|---|---|---|
| Original tone value | 0-63 | 64-127 | 128-191 | 192-255 |
| Converted tone value | 0 | 85 | 170 | 255 |

**[Table 3]**

| | | | | | |
|---|---|---|---|---|---|
| Original tone value | 0-50 | 51-101 | 102-152 | 153-203 | 204-255 |
| Converted tone value | 0 | 76 | 127 | 178 | 255 |

In the posterization indicated in Table 1 to Table 3, the tone values (0 to 255) of the original image are equally divided into a plurality of sections (three sections in Table 1, four sections in Table 2, and five sections in Table 3). Among the plurality of sections obtained by the division, in the first section, 0 (that is, black) is assigned as the tone value obtained after posterization, and, in the last section, 255 (that is, white) is assigned as the tone value obtained after the posterization. In the remaining sections, an intermediate tone value is assigned for each section.

The posterization is performed by inputting the number of tones to be obtained in the change, in the operation unit 4 shown in FIG. 1. For example, in order to obtain the 3 tone image, the number of tones=3 is inputted in the image processing unit 5 through the operation unit 4. The image processing unit 5 changes the number of tones of the original image to the number of tones inputted through the operation unit 4.

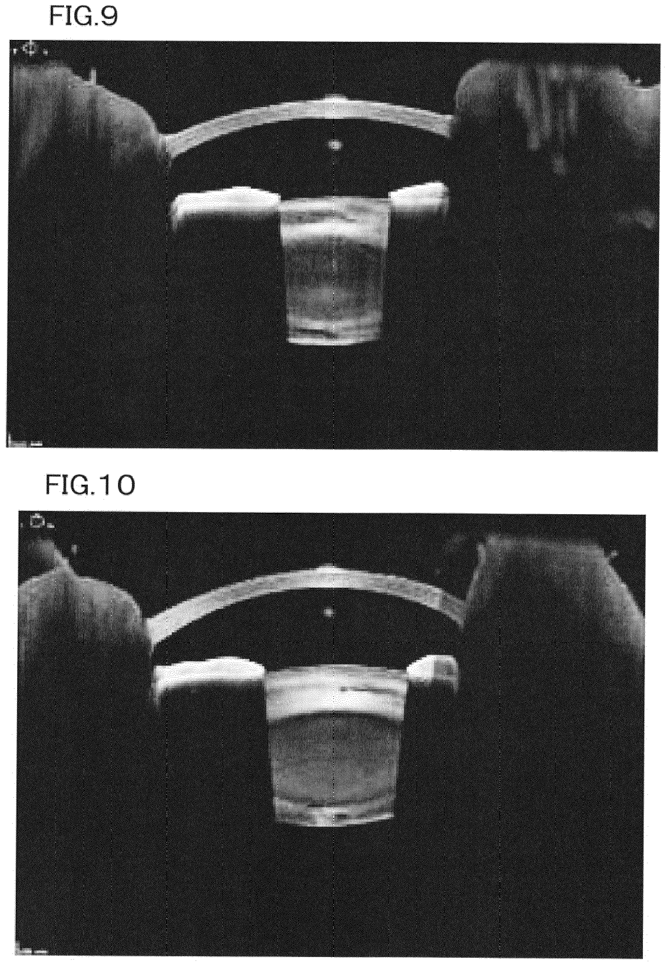

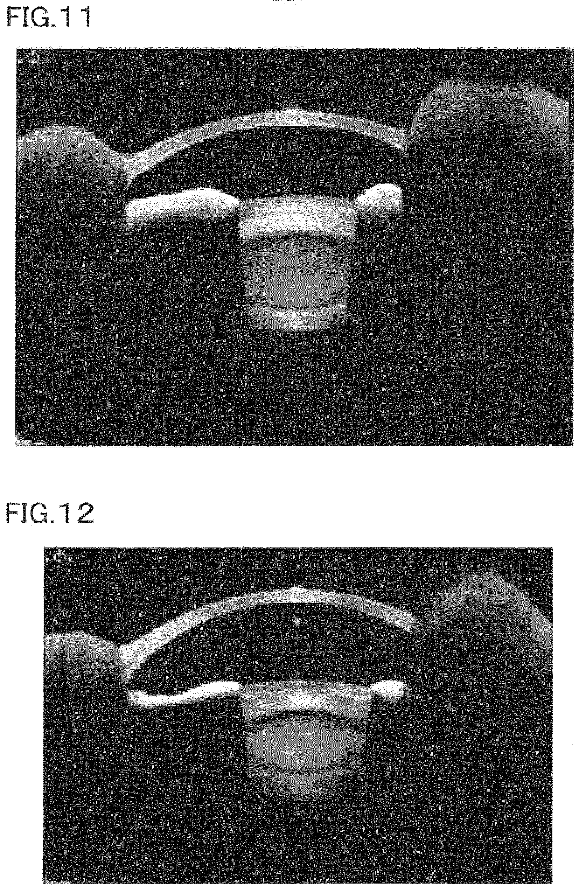

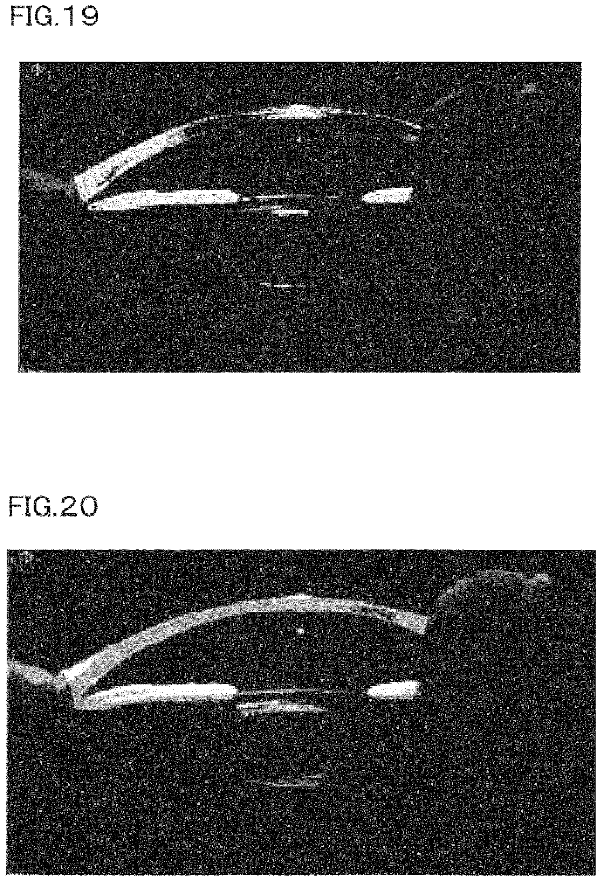

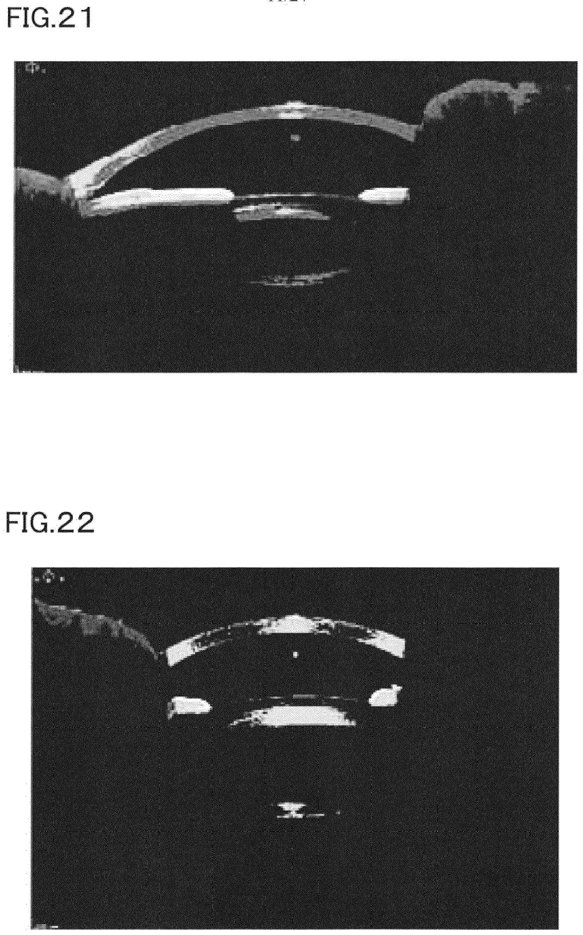

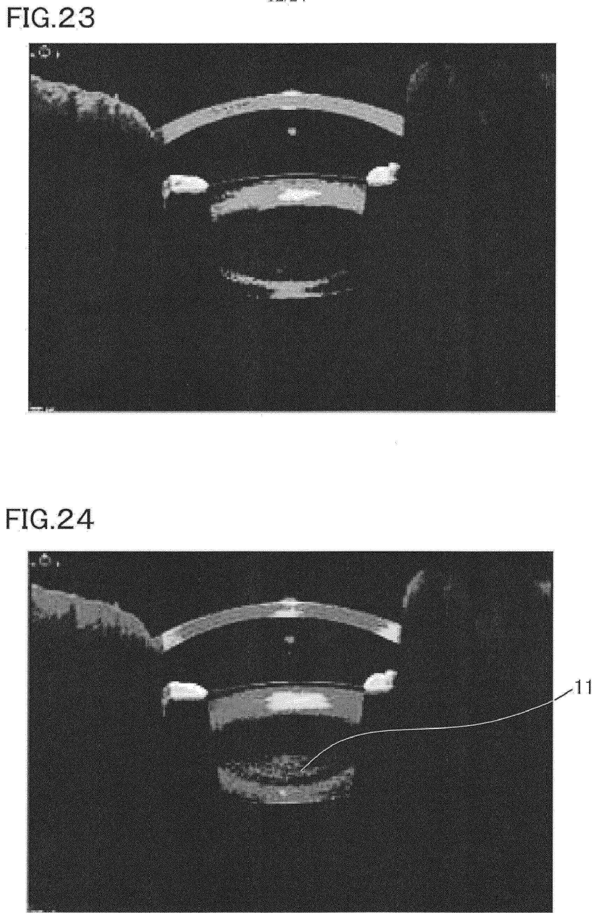

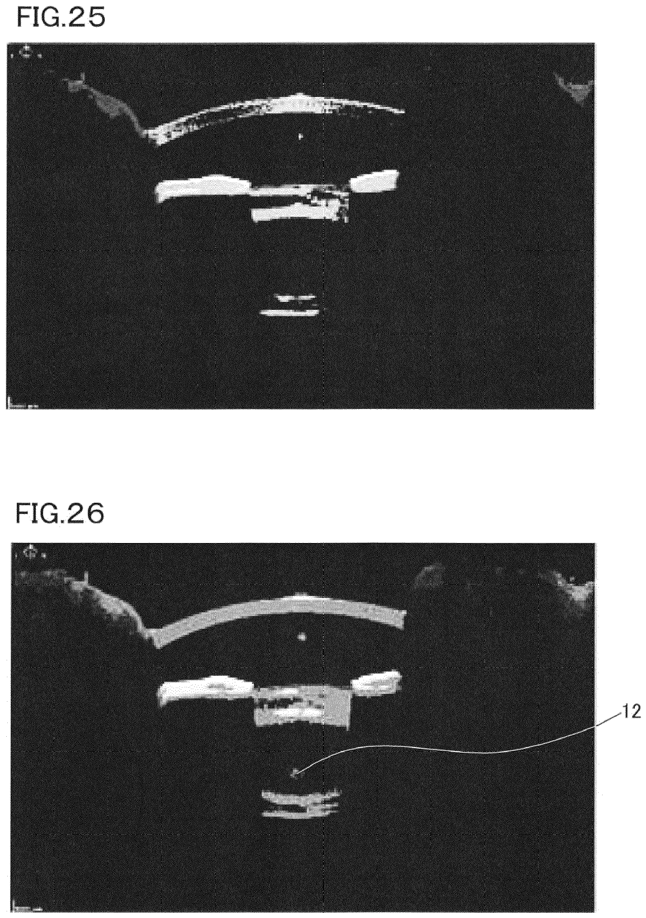

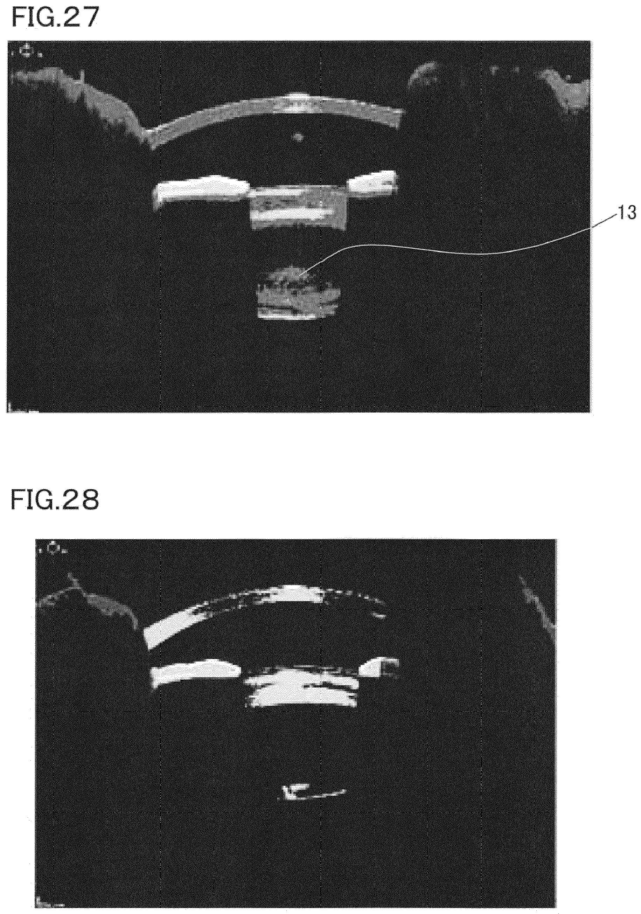

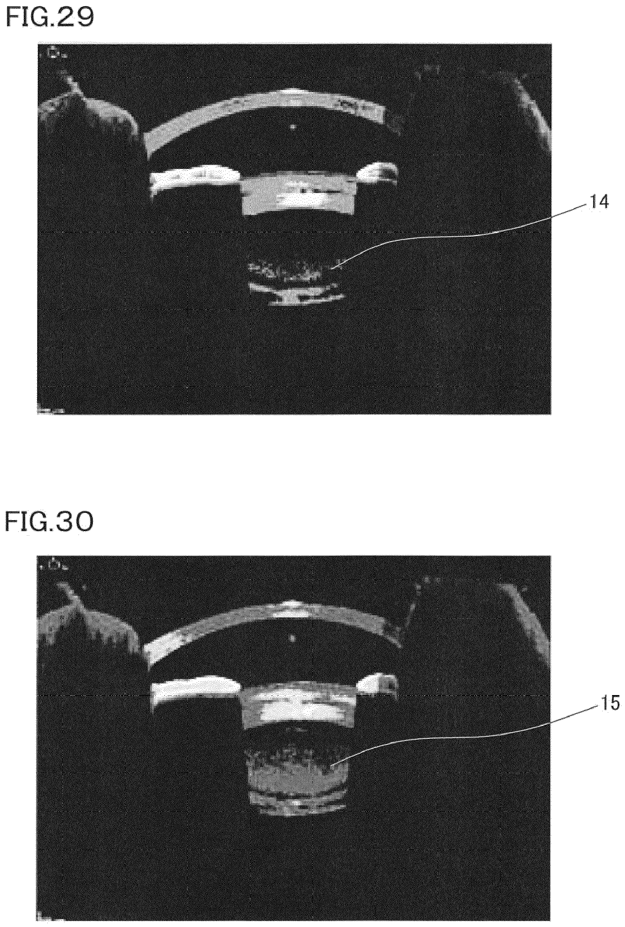

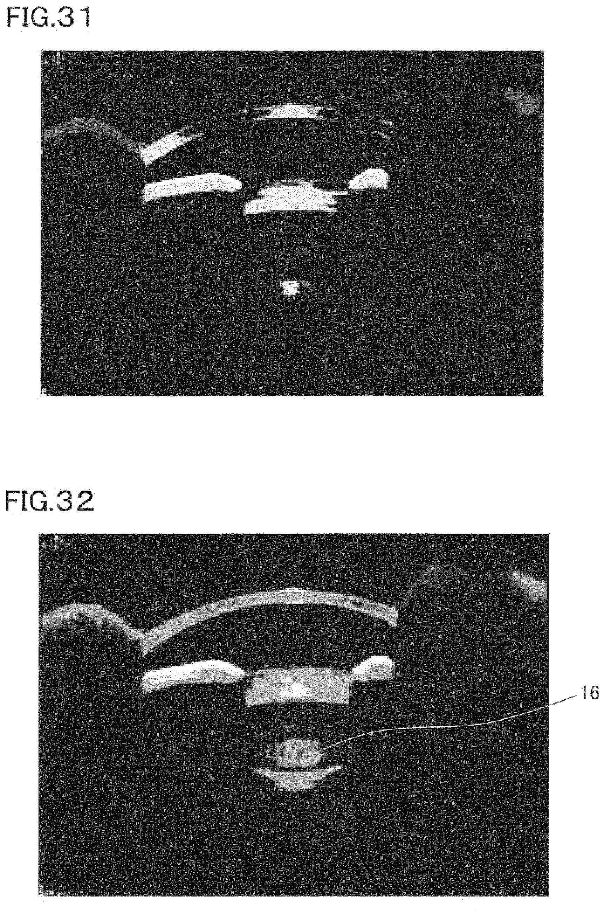

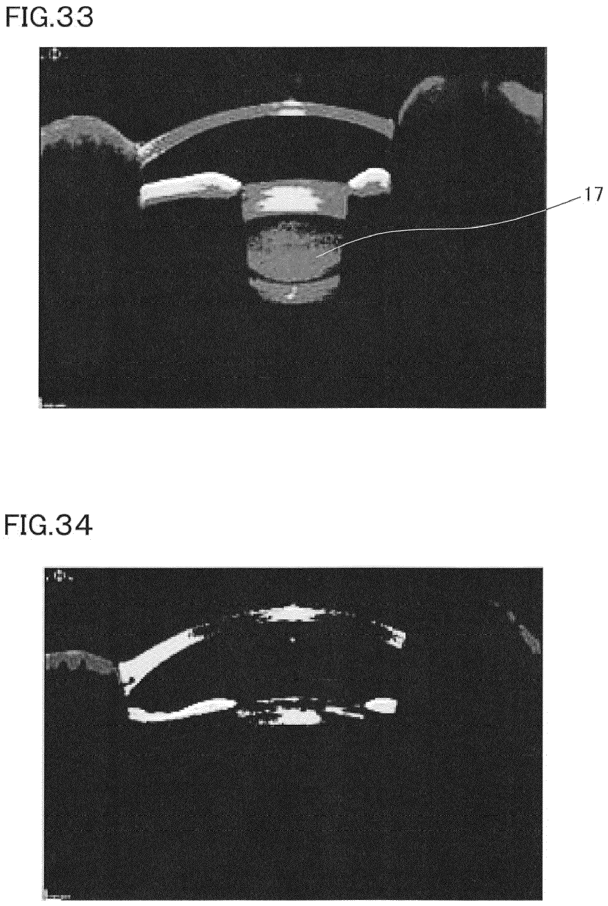

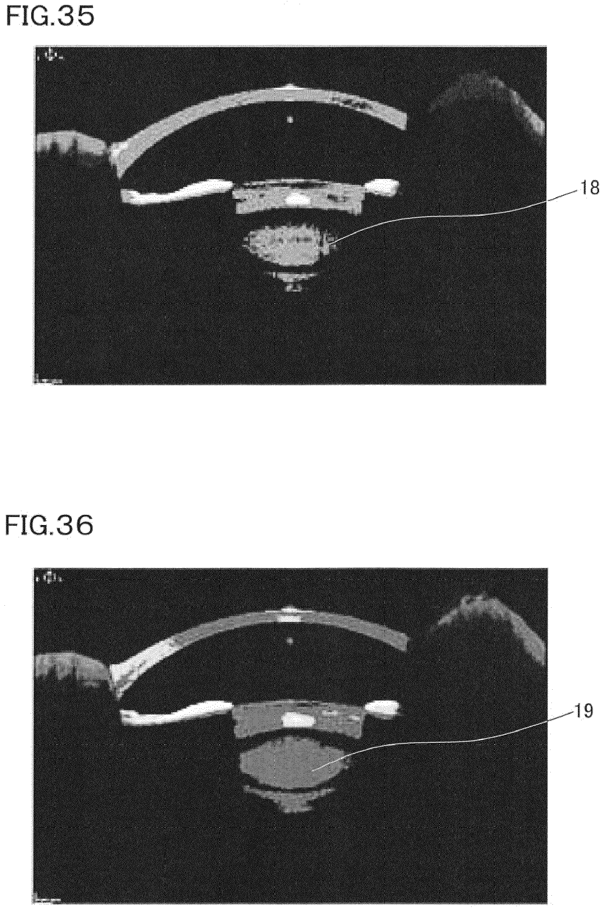

FIG. 19 to FIG. 21 illustrate examples in which the original image shown in FIG. 7 has been subjected to the posterization. FIG. 19 illustrates the 3 tone image, FIG. 20 illustrates the 4 tone image, and FIG. 21 illustrates the 5 tone image. FIG. 22 to FIG. 24 illustrate examples in which the original image shown in FIG. 8 has been subjected to the posterization. FIG. 22 illustrates the 3 tone image, FIG. 23 illustrates the 4 tone image, and FIG. 24 illustrates the 5 tone image. FIG. 25 to FIG. 27 illustrate examples in which the original image shown in FIG. 9 has been subjected to the posterization. FIG. 25 illustrates the 3 tone image, FIG. 26 illustrates the 4 tone image, and FIG. 27 illustrates the 5 tone image. FIG. 28 to FIG. 30 illustrate examples in which the original image shown in FIG. 10 has been subjected to the posterization. FIG. 28 illustrates the 3 tone image, FIG. 29 illustrates the 4 tone image, and FIG. 30 illustrates the 5 tone image. FIG. 31 to FIG. 33 illustrate examples in which the original image shown in FIG. 11 has been subjected to the posterization. FIG. 31 illustrates the 3 tone image, FIG. 32 illustrates the 4 tone image, and FIG. 33 illustrates the 5 tone image. FIG. 34 to FIG. 36 illustrate examples in which the original image shown in FIG. 12 has been subjected to the posterization. FIG. 34 illustrates the 3 tone image, FIG. 35 illustrates the 4 tone image, and FIG. 36 illustrates the 5 tone image.

Subsequently, a precondition for classifying crystalline lens nucleus states is set based on tone-changed images that are images having been subjected to the posterization in step S2 (S3). That is, the number of grades in classification and a criterion for each grade are set in S3. Specifically, each grade in the classification is set according to the procedure in FIG. 4. Each step in FIG. 4 is performed by a person such as a doctor.

In FIG. 4, a state of an image of the crystalline lens nucleus observed in the tone-changed image is firstly specified for each tone-changed image (S11). In this embodiment, whether or not an image of the crystalline lens nucleus is observed in the image (whether or not an observed image is present) is determined, or the shape or the size of the observed image is determined, to specify the state of the observed image. The shape or the size of the observed image is specified by, for example, determining whether the entirety or a part of the crystalline lens nucleus is observed, or determining, in a case where a part of the crystalline lens nucleus is observed, the shape of the observed image of the crystalline lens nucleus. In step S11, a region indicated by a color (that is, white or gray) other than black at the center portion (crystalline lens nucleus region) of the crystalline lens is specified as the observed image of the crystalline lens nucleus. As the state of the observed image, difference in depth of the white or gray color in the observed image is not taken into consideration.

Step S11 will be described by using the tone-changed images shown in FIG. 19 to FIG. 36 as an example. In the tone-changed images, shown in FIG. 19 to FIG. 21, obtained from the original image in FIG. 7, an image of the crystalline lens nucleus is not observed in any of the 3 tone image, the 4 tone image, and the 5 tone image. In the tone-changed images, shown in FIG. 22 to FIG. 24, obtained from the original image in FIG. 8, an image of the crystalline lens nucleus is not observed in the 3 tone image and the 4 tone image in FIG. 22 and FIG. 23, and an image 11 of the crystalline lens nucleus is observed in the 5 tone image in FIG. 24. The observed image 11 represents a part of the crystalline lens nucleus according to the size.

In the tone-changed images, shown in FIG. 25 to FIG. 27, obtained from the original image in FIG. 9, an image of the crystalline lens nucleus is not observed in the 3 tone image in FIG. 25, and images 12 and 13 of the crystalline lens nucleus are observed in the 4 tone image and the 5 tone image in FIG. 26 and FIG. 27. The observed images 12 and 13 each represent a part of the crystalline lens nucleus according to the size, and, more specifically, represent spot-shaped images. The "spot-shaped" represents a shape having an aspect ratio closer to 1 than "band-shaped" described below, and represents a dot-like shape or a shape close to a dot.

In the tone-changed images, shown in FIG. 28 to FIG. 30, obtained from the original image in FIG. 10, an image of the crystalline lens nucleus is not observed in the 3 tone image in FIG. 28, and images 14, 15 of the crystalline lens nucleus are observed in the 4 tone image and the 5 tone image in FIG. 29 and FIG. 30. The observed images 14, 15 each represent a part of the crystalline lens nucleus according to the size, and, more specifically, represent band-shaped images. The "band-shaped" represent a shape elongated laterally or vertically as compared with the above-described "spot-shaped". The observed images 14, 15 are elongated in the lateral (left-right) direction.

In the tone-changed images, shown in FIG. 31 to FIG. 33, obtained from the original image in FIG. 11, an image of the crystalline lens nucleus is not observed in the 3 tone image in FIG. 31, and images 16, 17 of the crystalline lens nucleus are observed in the 4 tone image and the 5 tone image in FIG. 32 and FIG. 33. The observed image 16 in the 4 tone image represents a part of the crystalline lens nucleus according to the size. The observed image 17 in the 5 tone image represents the entirety of the crystalline lens nucleus according to the size.

In the tone-changed images, shown in FIG. 34 to FIG. 36, obtained from the original image in FIG. 12, an image of the crystalline lens nucleus is not observed in the 3 tone image in FIG. 34, and images 18, 19 of the crystalline lens nucleus are observed in the 4 tone image and the 5 tone image in FIG. 35 and FIG. 36. The observed images 18, 19 each represent the entirety of the crystalline lens nucleus according to the size.

Returning to FIG. 4, subsequently, the states of the observed images of the crystalline lens nuclei which have been specified in step S11 are classified into a plurality of grades (S12). Specifically, three images of the 3 tone image, the 4 tone image, and the 5 tone image which are obtained from one original image are set as one image set, and a combination of a state of the observed image of the crystalline lens nucleus in the 3 tone image, a state of the observed image of the crystalline lens nucleus in the 4 tone image, and a state of the observed image of the crystalline lens nucleus in the 5 tone image is confirmed for each image set. Commonality and difference among the combinations of a plurality of image sets are found and the combinations are classified into a plurality of grades. In this embodiment, for example, classification into 6 grades is performed as indicated in Table 4.

A first grade in Table 4 represents a grade in which an image of the crystalline lens nucleus is not observed in any of the 3 tone image, the 4 tone image, and the 5 tone image. The first grade corresponds to the image set of the images shown in FIG. 19 to FIG. 21.

A second grade in Table 4 represents a grade in which an image of the crystalline lens nucleus is not observed in the 3 tone image and the 4 tone image, and an image of a part of the crystalline lens nucleus is observed in the 5 tone image. The second grade corresponds to the image set of the images shown in FIG. 22 to FIG. 24.

A third grade in Table 4 represents a grade in which an image of the crystalline lens nucleus is not observed in the 3 tone image, and images of the crystalline lens nucleus are observed as spot-shaped images in the 4 tone image and the 5 tone image. The third grade corresponds to the image set of the images shown in FIG. 25 to FIG. 27.

A fourth grade in Table 4 represents a grade in which an image of the crystalline lens nucleus is not observed in the 3 tone image, and images of the crystalline lens nucleus are observed as band-shaped images in the 4 tone image and the 5 tone image. The fourth grade corresponds to the image set of the images shown in FIG. 28 to FIG. 30.

A fifth grade in Table 4 represents a grade in which an image of the crystalline lens nucleus is not observed in the 3 tone image, an image of a part of the crystalline lens nucleus is observed in the 4 tone image, and an image of the entirety of the crystalline lens nucleus is observed in the 5 tone image. The fifth grade corresponds to the image set of the images shown in FIG. 31 to FIG. 33.

A sixth grade in Table 4 represents a grade in which an image of the crystalline lens nucleus is not observed in the 3 tone image, and an image of the entirety of the crystalline lens nucleus is observed in each of the 4 tone image and the 5 tone image. The sixth grade corresponds to the image set of the images shown in FIG. 34 to FIG. 36.

**[Table 4]**

| Classification grade | Nucleus classification number | Criterion |
|---|---|---|
| 1 | 1 | Nucleus is not observed in any of 3 tone image, 4 tone image, and 5 tone image |
| 2 | 2 | Nucleus is not observed in 3 tone image and 4 tone image A part of nucleus is observed in 5 tone image |
| 3 | 3a | Nucleus is not observed in 3 tone image Nucleus is observed as spot-shaped images in 4 tone image and 5 tone image |
| 4 | 3b | Nucleus is not observed in 3 tone image Nucleus is observed as band-shaped images in 4 tone image and 5 tone image |
| 5 | 4 | Nucleus is not observed in 3 tone image A part of nucleus is observed in 4 tone image The entirety of nucleus is observed in 5 tone image |
| 6 | 5 | Nucleus is not observed in 3 tone image The entirety of nucleus is observed in 4 tone image and 5 tone image |

Crystalline lens cells are densified and pressure is applied to the crystalline lens cells according to aging, so that the crystalline lens nucleus is hardened. In a case where the crystalline lens nucleus is hardened, an intensity of light (reflected light) generated by reflecting measurement light of the optical coherence tomograph 2 by the inside of the crystalline lens nucleus is increased, so that the observed image of the crystalline lens nucleus is easily formed in a tomographic image of the anterior segment. In this case, the greater the number of the hardened portions of the crystalline lens nucleus is, the larger the observed image is. The harder each portion of the crystalline lens nucleus is, the more easily the image is formed, even in the tone-changed image having a reduced tone. The crystalline lens nucleus becomes whitish or yellowish according to aging. In other words, transparency is lowered. The depth of white or yellow color, opacity, and hardness of the crystalline lens nucleus correlate with each other. That is, the harder the crystalline lens nucleus is, the deeper the color is and the higher the opacity is. In other words, the deeper the color is and the higher the opacity is, the harder the crystalline lens nucleus is.

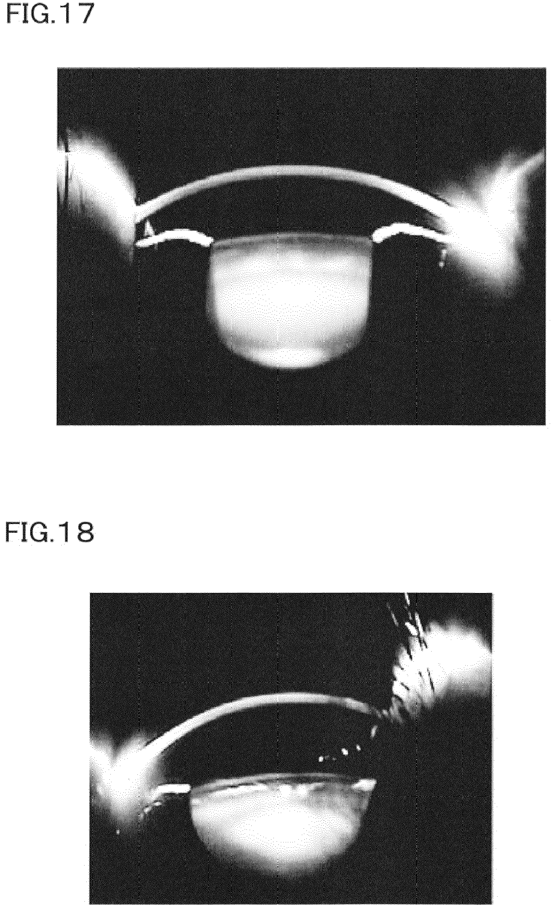

As described above, in a plurality of the tone-changed images (3 tone image, 4 tone image, and 5 tone image), it can be considered that the greater the number of observed images of the crystalline lens nucleus is or the larger an image of the observed crystalline lens nucleus is, the higher the hardness, the color depth, or the opacity (cloudiness) of the crystalline lens nucleus is. In Table 4, it can be considered that the hardness, color depth, or opacity of the crystalline lens nucleus is increased in the order of the first grade→the second grade→the third grade→the fourth grade→the fifth grade→the sixth grade. This is almost the same as the tendency, in the color depth or opacity, of the crystalline lens nuclei in the images taken by the slit-lamp microscope as shown in FIG. 13 to FIG. 18. That is, comparison among the images in FIG. 13 to FIG. 18 indicates that the color of the crystalline lens center portion (crystalline lens nucleus), shown in FIG. 13, corresponding to the first grade in Table 4 is lightest, and the color of the crystalline lens center portion (crystalline lens nucleus), shown in FIG. 18, corresponding to the sixth grade in Table 4 is deepest.

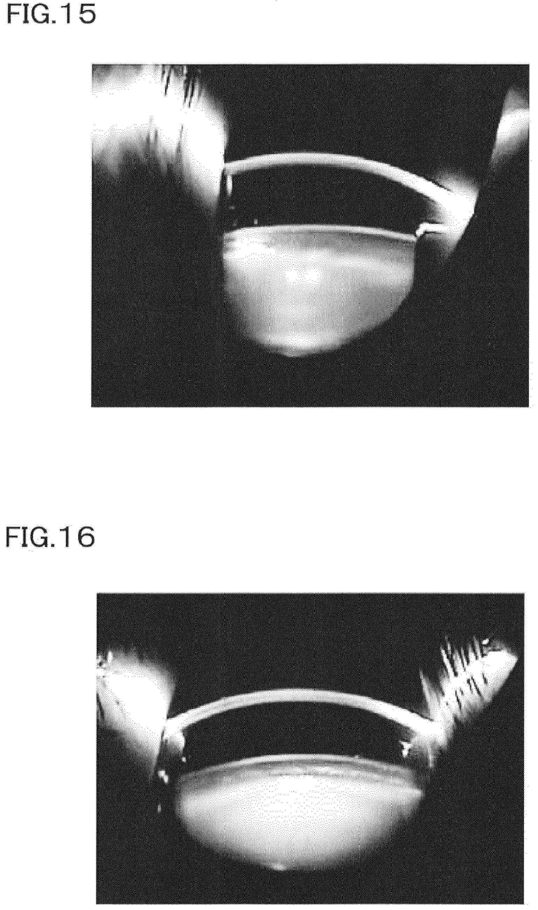

More specifically, the image, in FIG. 13, corresponding to the first grade (classification number "1" described below) in Table 4 corresponds to the second standard NC2 among six standards NC1 to NC6, listed in Fig. 5 of the above-described "The Lens Opacities Classification System III", for a color or opacity of a crystalline lens nucleus. The image, in FIG. 14, corresponding to the second grade (classification number "2" described below) in Table 4 corresponds to the third standard NC3 among the six standards NC1 to NC6 described above. The image, in FIG. 15, corresponding to the third grade (classification number "3a" described below) in Table 4 corresponds to the third standard NC3 among the six standards NC1 to NC6 described above. The image, in FIG. 16, corresponding to the fourth grade (classification number "3b" described below) in Table 4 corresponds to the fourth standard NC4 among the six standards NC1 to NC6 described above. The image, in FIG. 17, corresponding to the fifth grade (classification number "4" described below) in Table 4 corresponds to the fifth standard NC5 among the six standards NC1 to NC6 described above. The image, in FIG. 18, corresponding to the sixth grade (classification number "5" described below) in Table 4 corresponds to the sixth standard NC6 among the six standards NC1 to NC6 described above. Thus, the grades in Table 4 correlate with the classification (NC1 to NC6) of crystalline lens nuclei in the above-described "The Lens Opacities Classification System III".

Subsequently, a classification number corresponding to a state (hardness) of a crystalline lens nucleus is assigned to each of the grades classified in step S12 (S13). Table 4 indicates an example in which, as the classification numbers, "1" is assigned to the first grade, "2" is assigned to the second grade, "3a" is assigned to the third grade, "3b" is assigned to the fourth grade, "4" is assigned to the fifth grade, and "5" is assigned to the sixth grade. The third grade and the fourth grade are the same in that the image of a part of the crystalline lens nucleus is observed in each of the 4 tone image and the 5 tone image. Therefore, the same number "3" is assigned as the classification numbers to the third grade and the fourth grade, and alphabets "a" and "b" representing a spot-shaped observed image and a band-shaped observed image, respectively, are added to the numbers. The band-shaped observed images 14, 15 (see FIG. 29, FIG. 30) of the fourth grade "3b" are slightly larger than the spot-shaped observed images 12 and 13 (see FIG. 26, FIG. 27) of the third grade "3a". Therefore, the fourth grade "3b" is slightly higher than the third grade "3a" in terms of hardness of a crystalline lens nucleus.

Thus, the condition (Table 4) as a precondition (criteria) for classifying hardnesses of the crystalline lens nuclei can be obtained. Table 4 indicates correspondence between each of a plurality of grades into which the states (hardnesses) of the crystalline lens nuclei are classified, and a combination of states of the observed images of the crystalline lens nucleus in the tone-changed images having 3 tones, 4 tones, and 5 tones. The correspondence indicated in Table 4 is considered to be widely used by persons other than the person who has derived Table 4. Furthermore, the reference tone-changed images having 3 tones, 4 tones, and 5 tones may be set as a criterion for each grade in Table 4 in addition to or instead of the conditions expressed by characters. The images shown in, for example, FIG. 19 to FIG. 21 are set as reference images for the first grade. The images shown in, for example, FIG. 22 to FIG. 24 are set as reference images for the second grade. The images shown in, for example, FIG. 25 to FIG. 27 are set as reference images for the third grade. The images shown in, for example, FIG. 28 to FIG. 30 are set as reference images for the fourth grade. The images shown in, for example, FIG. 31 to FIG. 33 are set as reference images for the fifth grade. The images shown in, for example, FIG. 34 to FIG. 36 are set as reference images for the sixth grade.

A procedure shown in FIG. 5, FIG. 6 for determining one, of the grades in Table 4, corresponding to a state (hardness) of a crystalline lens nucleus in a crystalline lens, will be described. In FIG. 5, an anterior segment image to be determined is firstly obtained (S21). The anterior segment image to be obtained is an image of an eye diagnosed as having a cataract, and is also a gray scale tomographic image taken by optical coherence tomography (the optical coherence tomograph 2).

Subsequently, the image processing unit 5 reads the anterior segment image obtained in step S21, and the image processing unit 5 performs a process (posterization) of lowering the number of tones of the anterior segment image (S22). The posterization in step S22 is the same as that in step S2 shown in FIG. 3. That is, a tone value of each pixel of the original image that is the anterior segment image obtained in step S21 is converted as indicated in Table 1 to Table 3, and the 3 tone image, the 4 tone image, and the 5 tone image are obtained. The image processing unit 5 presents the 3 tone image, the 4 tone image, and the 5 tone image (presenting step).

Subsequently, the state of the crystalline lens nucleus of the crystalline lens to be determined is classified based on the 3 tone image, the 4 tone image, and the 5 tone image which have been obtained in step S22 (S23). Specifically, the state of the crystalline lens nucleus is classified according to the procedure in FIG. 6. Each step in FIG. 6 is performed by a person such as a doctor.

In FIG. 6, a state of an image of the crystalline lens nucleus observed in the tone-changed image is firstly specified for each tone-changed image obtained in step S22 in FIG. 5 (S31). Step S31 is the same as step S11 in FIG. 4.

Subsequently, a combination of a state of the observed image in the 3 tone image, a state of the observed image in the 4 tone image, and a state of the observed image in the 5 tone image as have been specified in step S31 is compared with the correspondence indicated in Table 4, to determine a grade, in Table 4, corresponding to the state of the crystalline lens nucleus of the crystalline lens to be determined (S32). The classification number corresponding to the determined grade is assigned as the state of the crystalline lens nucleus. For example, in a case where an image of a nucleus is not observed in the 3 tone image, an image of a part of the nucleus is observed in the 4 tone image, and an image of the entirety of the nucleus is observed in the 5 tone image, the grade is determined as corresponding to the fifth grade in Table 4, and "4" is assigned as the nucleus classification number.

Thereafter, a cataract surgery method is determined, ultrasonic output in the phacoemulsification and aspiration is adjusted, or a person who performs the cataract surgery is determined based on the determined hardness of the crystalline lens nucleus. Step S32 is equivalent to determination of a color depth or opacity (cloudiness) of the crystalline lens nucleus.

As described above, in this embodiment, the posterization of a gray scale anterior segment image obtained by optical coherence tomography is performed. Therefore, a state of the crystalline lens nucleus in the image can be emphasized. Thus, the crystalline lens data (anterior segment image data) that allows the crystalline lens nucleus state to be easily determined can be obtained. Data having high objectivity or reproducibility can be obtained as data that allows the crystalline lens nucleus state to be easily determined based on the crystalline lens data, and that indicates the determination result of the crystalline lens nucleus state. That is, in a case where, for example, an evaluator A determines, as classification number "2", a nucleus state of a crystalline lens X to be determined, the same classification number "2" may highly likely be assigned when the same evaluator A determines a nucleus state of the same crystalline lens X to be determined at another time, or another evaluator B determines a nucleus state of the same crystalline lens X to be determined. Furthermore, since the crystalline lens nucleus state is determined based on the gray scale tone-changed image, a color other than black, white, and gray need not be determined, whereby the determination is facilitated.

In determination (classification) of the crystalline lens nucleus state, presence or absence, the shape, or the size (whether the observed image of a nucleus is the entire image or a partial image, whether the observed image is a spot-shaped image or a band-shaped image) of the observed image of the crystalline lens nucleus is determined, and the number of the tone-changed images in which the image of the crystalline lens nucleus can be observed or the number of tones of each of the tone-changed images, or the number of the tone-changed images in which the image thereof cannot be observed or the number of tones of each of the tone-changed images is determined without determining difference among colors of the observed images of the crystalline lens nuclei, whereby a crystalline lens nucleus state can be easily determined.

Meanwhile, in a case where a color or transparency of a crystalline lens nucleus is determined as the crystalline lens nucleus state in a color image obtained by a slit-lamp microscope or the like, determination of subtle differences in color or transparency is difficult. For example, in the images, shown in FIG. 13 to FIG. 18, obtained by the slit-lamp microscope, although difference in color between the image in FIG. 13 and the image in FIG. 18 can be determined, determination of difference in color between the image in FIG. 14 and the image in FIG. 15, and determination of difference in color between the image in FIG. 16 and the image in FIG. 17 are difficult. More specifically, both the image in FIG. 14 and the image in FIG. 15 are determined as the third grade NC3 in the crystalline lens nucleus classification according to the above-described "The Lens Opacities Classification System III". Meanwhile, in this embodiment, the image in FIG. 14 is classified as the second grade (classification number "2") in Table 4, and the image in FIG. 15 is classified as the third grade (classification number "3a") in Table 4. That is, in this embodiment, the image in FIG. 14 and the image in FIG. 15 can be classified as different grades, respectively.

Furthermore, in this embodiment, a crystalline lens nucleus state is classified based on a combination of a plurality of tone-changed images (the tone-changed images having 3 tones, 4 tones, and 5 tones). Therefore, the number of grades in classification can be increased as compared with classification based on a single image, and an amount of information to be read from one image can be reduced.

Moreover, since tones of the tone-changed image are 3 tones, 4 tones, or 5 tones, an image of a crystalline lens nucleus can be distinguished from an image of a crystalline lens cortex, and a crystalline lens nucleus state can be easily or correctly determined.

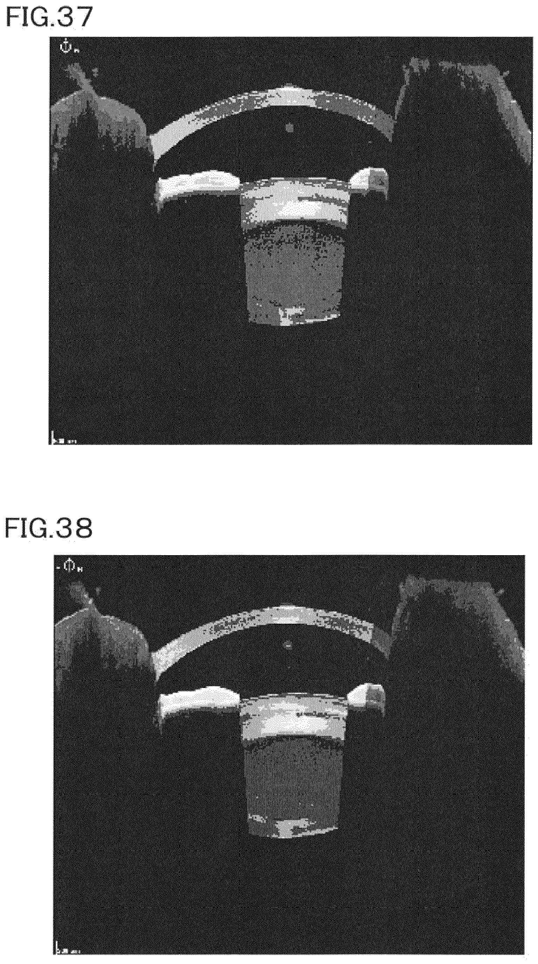

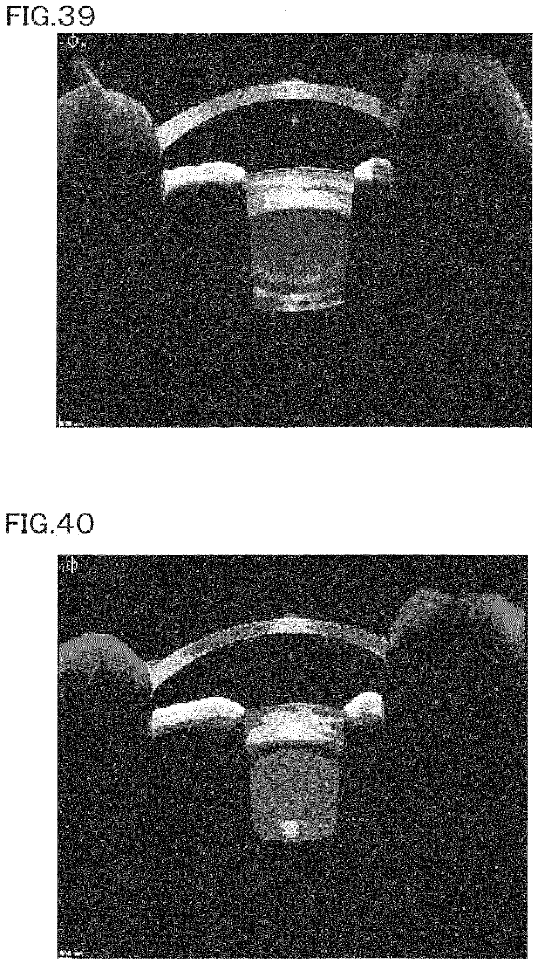

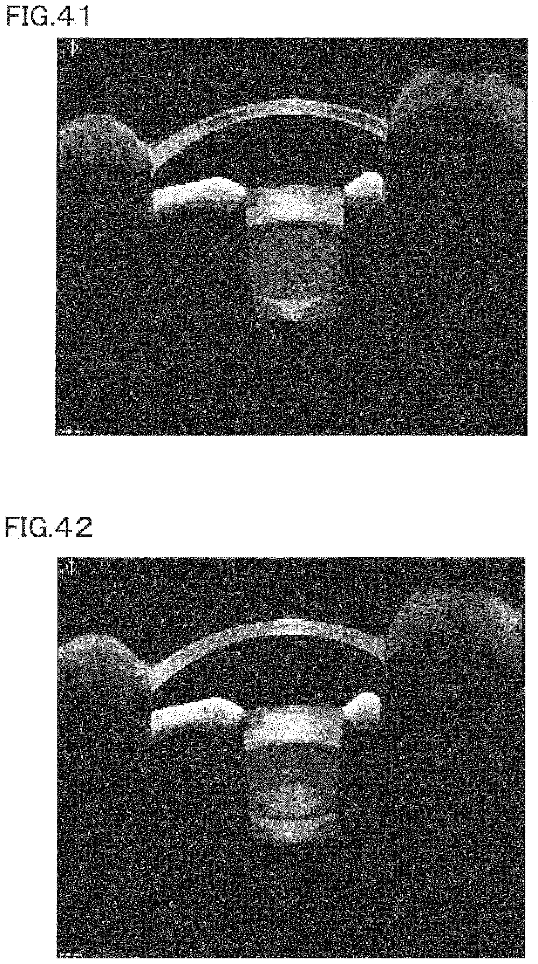

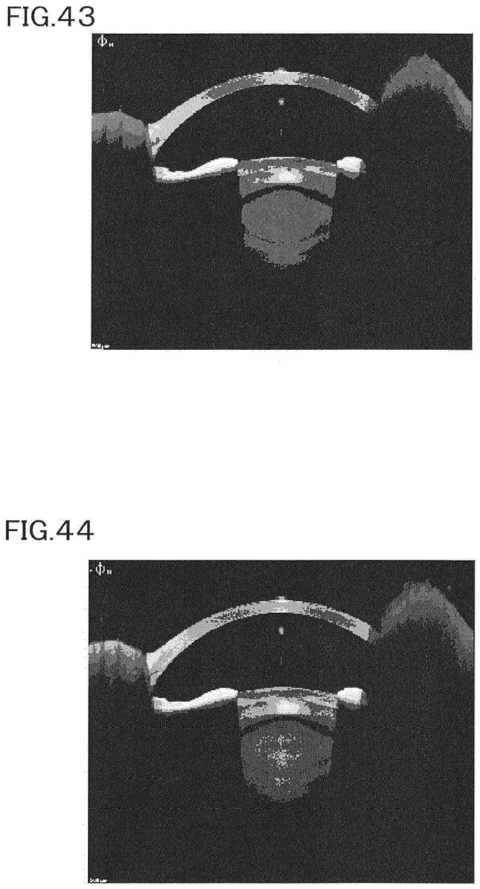

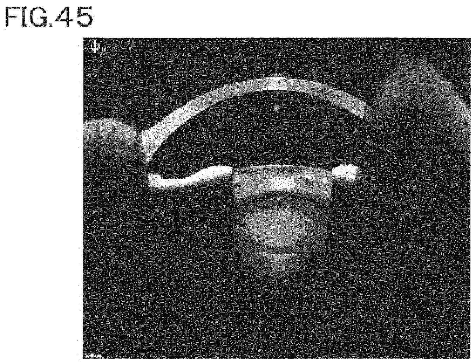

Meanwhile, FIG. 37 to FIG. 45 illustrate examples of tone-changed images having the number of tones changed to 6 tones, 7 tones, and 8 tones. FIG. 37 to FIG. 39 illustrate images obtained by subjecting the original image in FIG. 10 to the posterization. FIG. 37 illustrates a 6 tone image. FIG. 38 illustrates a 7 tone image. FIG. 39 illustrates an 8 tone image. FIG. 40 to FIG. 42 illustrate images obtained by subjecting the original image in FIG. 11 to the posterization. FIG. 40 illustrates a 6 tone image. FIG. 41 illustrates a 7 tone image. FIG. 42 illustrates an 8 tone image. FIG. 43 to FIG. 45 illustrate images obtained by subjecting the original image in FIG. 12 to the posterization. FIG. 43 illustrates a 6 tone image. FIG. 44 illustrates a 7 tone image. FIG. 45 illustrates an 8 tone image. As illustrated in FIG. 37 to FIG. 45, in a case where the number of tones is set to be not less than 6 in the posterization, an image of a crystalline lens nucleus and an image of a crystalline lens cortex are blended. In other words, a boundary between the image of the crystalline lens nucleus and the image of the crystalline lens cortex becomes unclear, and determination of a crystalline lens nucleus state becomes difficult.

As indicated in Table 4, although an image of a crystalline lens nucleus is not observed in the 3 tone image at any grade, in a case where the 3 tone image is included in the classification criterion for each grade, the 4 tone image and the 5 tone image can be determined based on the 3 tone image, and presence or absence of an observed image of a crystalline lens nucleus and a state of the observed image can be easily determined in the 4 tone image and the 5 tone image.

### (Second embodiment)

Next, a second embodiment of this disclosure will be described. In the first embodiment, a person determines (classifies the grade of) a crystalline lens nucleus state. In this embodiment, the determination is performed by a device. The configuration of this embodiment includes the same ophthalmological examination system 1 as that in the first embodiment as shown in FIG. 1. Data (for example, data in Table 4) in which a crystalline lens nucleus state and a state of an image of a crystalline lens nucleus in an anterior segment image are associated with each other, and reference data (for example, as shown in FIG. 19 to FIG. 36, an anterior segment image in which an image of a crystalline lens nucleus is not observed, an anterior segment image in which an image of a crystalline lens nucleus is spot-shaped, an anterior segment image in which an image of a crystalline lens nucleus is band-shaped, and an anterior segment image in which the entire image of a crystalline lens nucleus is observed) for specifying a state of an image of a crystalline lens nucleus, are stored in the memory 7 of the image processing unit 5.

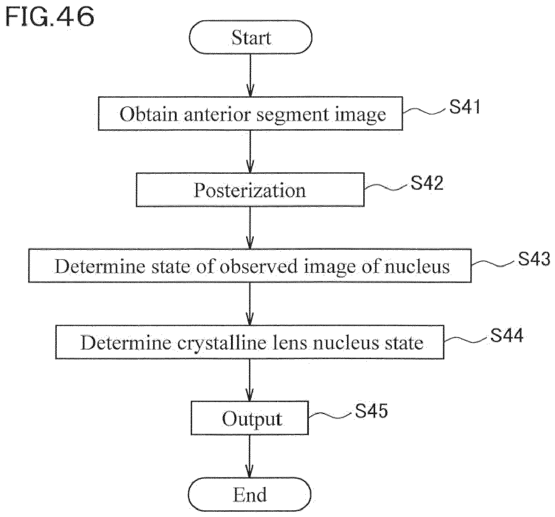

The controller 6 of the image processing unit 5 performs, for example, a process shown in FIG. 46 to determine a crystalline lens nucleus state of a crystalline lens to be determined. The process shown in FIG. 46 will be described below. The program 8 stored in the memory 7 is read to perform the process shown in FIG. 46.

By starting the process shown in FIG. 46, the controller 6 obtains an anterior segment image to be determined (S41). The anterior segment image to be obtained is an image of an eye diagnosed as having a cataract, and is also a gray scale tomographic image taken by optical coherence tomography (the optical coherence tomograph 2). Specifically, for example, the controller 6 operates to read an anterior segment image indicated through an operation on the operation unit 4, from a storage unit (for example, the memory of the optical coherence tomograph 2, the memory 7 of the image processing unit 5, an external memory, or the like) in which anterior segment images taken by optical coherence tomography are stored.

Subsequently, the posterization of the anterior segment image obtained in step S41 is performed (S42). The posterization is the same as that in step S2 in FIG. 3. That is, a tone value of each pixel in the original image that is the anterior segment image obtained in step S41 is converted as indicated in Table 1 to Table 3, to obtain a 3 tone image, a 4 tone image, and a 5 tone image.

Subsequently, a state of an observed image of a crystalline lens nucleus in each of the tone-changed images (the 3 tone image, the 4 tone image, and the 5 tone image) obtained in step S42 is determined (S43). Specifically, for example, as shown in FIG. 19 to FIG. 36, an anterior segment image is previously stored as a reference image in the memory 7 for each state of the observed image of the crystalline lens nucleus. Data indicating correspondence between each reference image and a state of an observed image of a crystalline lens nucleus is also stored in the memory 7. The tone-changed image and each reference image stored in the memory 7 are compared with each other for each of the tone-changed images having 3 tones, 4 tones, and 5 tones as obtained in step S42, to specify a reference image approximate to the tone-changed image. The state of the observed image of the crystalline lens nucleus in the specified approximate reference image is specified based on the above-described correspondence. The specified state of the observed image is determined as a state of the observed image of the crystalline lens nucleus in the tone-changed image to be determined.

Subsequently, a combination of the states of the observed images of the crystalline lens nucleus in the tone-changed images (the 3 tone image, the 4 tone image, and the 5 tone image) determined in step S43, is compared with data (for example, data in Table 4), stored in the memory 7, in which a combination of states of the observed images of the crystalline lens nucleus is associated with a state of the crystalline lens nucleus, to determine a state of the crystalline lens nucleus in the crystalline lens to be determined (S44). For example, a grade, in the first to the sixth grades indicated in Table 4, corresponding to the state of the crystalline lens nucleus is determined.

Subsequently, the display unit 3 or the like is caused to output the crystalline lens nucleus state determined in step S44 (S45). For example, in a case where one of the grades indicated in Table 4 is determined as the crystalline lens nucleus state in step S44, the nucleus classification number (see Table 4) corresponding to the determined grade is outputted in step S45. Thus, an evaluator such as a doctor is allowed to know the hardness of the crystalline lens nucleus.

Thus, in this embodiment, the crystalline lens nucleus state is determined by the device, whereby determination result having higher objectivity or reproducibility can be obtained.

This disclosure is not limited to the above-described embodiments, and various modifications can be devised. For example, although the nucleus state is determined based on the tone-changed images having 3 tones, 4 tones, and 5 tones in the above-described embodiments, since an image of a nucleus is not observed in the 3 tone image in any nucleus state, the nucleus state may be determined based on only the 4 tone image and the 5 tone image without using the 3 tone image.

In the above-described embodiments, the nucleus states are classified into six grades based on the three tone-changed images (the 3 tone image, the 4 tone image, and the 5 tone image). However, the nucleus states may be classified into 7 or more grades by taking into consideration an image having other tones in addition to the 3 tone image, the 4 tone image, and the 5 tone image, or by more minutely determining a state of an observed image of a crystalline lens nucleus in each tone-changed image. In contrast thereto, the nucleus states may be classified into five or less grades by reducing the number of the tone-changed images to be taken into consideration, or more roughly determining a state of an observed image of a crystalline lens nucleus in the tone-changed image. For example, the nucleus states may be classified into two grades by determining whether or not the entire image of a nucleus is observed in the 5 tone image by using the 5 tone image only. In this case, in a case where the entire image of a nucleus is observed in the 5 tone image, the hardness of the nucleus may be determined as being "high", and, in a case where the entire image of a nucleus is not observed (only a part of the nucleus is observed or no nucleus is observed), the hardness of the nucleus may be determined as being "low".

In step S1 in FIG. 3, step S21 in FIG. 5,or step 41 in FIG. 46, a gray scale anterior segment image taken by optical coherence tomography is obtained. However, this disclosure is not limited thereto, and a gray scale anterior segment image taken by a technique other than optical coherence tomography may be obtained. Furthermore, a color image taken by optical coherence tomography or by a technique other than optical coherence tomography may be obtained to convert the color image to a gray scale image before the posterization. Moreover, a color image of an anterior segment is obtained to perform the posterization of the color image as it is, whereby a crystalline lens nucleus state may be determined based on the color tone-changed image.

In Table 4, "1", "2", "3a", "3b", "4", and "5" are assigned as the nucleus classification numbers. However, other characters may be assigned.

In the above-described embodiments, a state of a crystalline lens nucleus is determined. However, a state of a crystalline lens site (crystalline lens cortex, crystalline lens capsule, or the like) other than a crystalline lens nucleus may be determined based on an image having been subjected to the posterization. Alternatively, a state of a crystalline lens may be determined as a whole without discriminating the crystalline lens sites (crystalline lens nucleus, crystalline lens cortex, and the like).

In the above-described embodiments, the number of tones in the original image is 256. However, the number of tones may be other than 256.

In the above-described embodiments, a state of a crystalline lens nucleus diagnosed as a cataract is determined. However, an anterior segment image of an eye that is unclear as to whether or not the eye has a disease such as a cataract, may be obtained to perform the posterization of the anterior segment image of the eye, whereby a state of the crystalline lens nucleus, presence or absence of a disease of the crystalline lens, or presence or absence of injury to the crystalline lens may be determined based on the image having been subjected to the posterization.

In the above-described embodiments, step S1 in FIG. 3, step S21 in FIG. 5, and step S41 in FIG. 46 correspond to an obtaining step. Step S2 in FIG. 3, step S22 in FIG. 5, and step S42 in FIG. 46 correspond to a tone changing step. Step S3 in FIG. 3 corresponds to a setting step. Step S23 in FIG. 5 and steps S43 and S44 in FIG. 46 correspond to a determination step. The image processing unit 5 in FIG. 1 corresponds to an ophthalmologic device. The controller 6, in the image processing unit 5, which performs step S41 in FIG. 46 corresponds to an obtaining portion. The controller 6, in the image processing unit 5, which performs step S42 in FIG. 46 corresponds to a tone changing portion. The controller 6, in the image processing unit 5, which performs step S43 and step S44 in FIG. 46 corresponds to a determination portion. The program 8 in FIG. 1 corresponds to a program for the ophthalmologic device.

### DESCRIPTION OF THE REFERENCE CHARACTERS

- 1: ophthalmological examination system
- 2: optical coherence tomograph
- 3: display unit
- 4: operation unit
- 5: image processing unit
- 6: controller
- 7: memory
- 8: program

## Claims

1. A crystalline lens data obtaining method comprising:
an obtaining step (S1,S21,S41) of obtaining an image of a crystalline lens; and
a tone changing step (S2,S22,S42) of lowering the number of tones of an original image that is the image obtained in the obtaining step (S1,S21,S41).

2. The crystalline lens data obtaining method according to claim 1, wherein the original image represented by a gray scale is obtained in the obtaining step (S1,S21,S41).

3. The crystalline lens data obtaining method according to claim 1 or 2, wherein the original image is obtained by optical coherence tomography in the obtaining step (S1,S21,S41).

4. The crystalline lens data obtaining method according to any one of claims 1 to 3, wherein
an image having a tone changed in the tone changing step (S2,S22,S42) is a tone-changed image, and
a plurality of the tone-changed images having different numbers of tones are obtained from the one original image in the tone changing step (S2,S22,S42).

5. The crystalline lens data obtaining method according to claim 4, wherein the tone-changed images having 4 tones and 5 tones are obtained from the one original image in the tone changing step (S2,S22,S42).

6. The crystalline lens data obtaining method according to any one of claims 1 to 5, comprising a setting step (S3) of setting a correspondence between each of a plurality of grades into which states of crystalline lens nuclei are classified, and a state of an image of a crystalline lens nucleus observed in the tone-changed image that is an image having a tone changed in the tone changing step (S2,S22,S42).

7. The crystalline lens data obtaining method according to any one of claims 1 to 6, wherein
a correspondence between each of a plurality of grades into which states of crystalline lens nuclei are classified, and a state of an image of a crystalline lens nucleus observed in the tone-changed image that is an image having a tone changed in the tone changing step (S2,S22,S42), is previously set, the crystalline lens data obtaining method comprising
a determination step (S23,S43,S44) of comparing the state of the tone-changed image obtained from a crystalline lens to be determined, with the correspondence, to determine one, of the grades, corresponding to the state of a nucleus of the crystalline lens to be determined.

8. The crystalline lens data obtaining method according to claim 6 or 7, wherein
a plurality of the tone-changed images having different numbers of tones are obtained from the one original image in the tone changing step (S2,S22,S42), and
the correspondence represents a relationship between each of the grades and a combination of the states of the plurality of the tone-changed images having the different numbers of tones.

9. The crystalline lens data obtaining method according to any one of claims 6 to 8, wherein the state represents a hardness of a crystalline lens nucleus.

10. The crystalline lens data obtaining method according to any one of claims 6 to 9, wherein the grades are set for classification according to presence or absence, a shape, or a size of an image of a crystalline lens nucleus observed in the tone-changed image.

11. The crystalline lens data obtaining method according to any one of claims 6 to 10, wherein the grades are set for classification according to whether or not an entire image of a crystalline lens nucleus is observed in the tone-changed image, whether or not an image of a crystalline lens nucleus observed in the tone-changed image is spot-shaped, or whether or not an image of a crystalline lens nucleus observed in the tone-changed image is band-shaped.

12. The crystalline lens data obtaining method according to any one of claims 6 to 11, wherein
a plurality of the tone-changed images having different numbers of tones are obtained from the one original image in the tone changing step (S2,S22,S42), and
the grades are set for classification according to the number of the tone-changed images in which an image of a crystalline lens nucleus is present or the number of tones of each of the tone-changed images, or the number of the tone-changed images in which an image of a crystalline lens nucleus is absent or the number of tones of each of the tone-changed images, among the plurality of the tone-changed images obtained from the one original image.

13. The crystalline lens data obtaining method according to any one of claims 1 to 12, wherein an image of a crystalline lens diagnosed as a cataract is obtained in the obtaining step (S1,S21,S41).

14. An ophthalmologic device (5) comprising:
an obtaining portion (S41,6) configured to obtain an image of a crystalline lens; and
a tone changing portion (S42,6) configured to lower the number of tones of an original image that is the image obtained by the obtaining portion (S41,6).

15. The ophthalmologic device (5) according to claim 14, further comprising a determination portion (S43,S44,6) configured to determine a state of a crystalline lens nucleus based on a tone-changed image that is an image having a tone changed by the tone changing portion (S42,6).

16. A program (8) for an ophthalmologic device stored therein, the program (8) causing a computer to function as:
an obtaining portion (S41,6) configured to obtain an image of a crystalline lens; and
a tone changing portion (S42,6) configured to lower the number of tones of an original image that is the image obtained by the obtaining portion (S41,6).

17. The program (8) for an ophthalmologic device according to claim 16, wherein the program (8) causing a computer to function as a determination portion (S43,S44,6) configured to determine a state of a crystalline lens nucleus based on a tone-changed image that is an image having a tone changed by the tone changing portion (S42,6).
